# EUROPEAN PATENT SPECIFICATION

(11) **EP 3 245 970 B1**
(45) Date of publication and mention of the grant of the patent: **27.03.2019**
(21) Application number: 17171424.9
(22) Date of filing: 16.05.2017
(51) Int. Cl.: A61B 18/10, A61B 18/12

(54) **ELECTROSURGICAL UNIT WITH MODULATED OUTPUT FOR RF ABLATION SURGICAL DEVICES**
ELEKTROCHIRURGISCHE EINHEIT MIT MODULIERTEM AUSGANG FÜR CHIRURGISCHE HF-ABLATIONSVORRICHTUNGEN
UNITÉ ÉLECTROCHIRURGICALE AYANT UNE SORTIE MODULÉE POUR DISPOSITIFS CHIRURGICAUX D'ABLATION RF

(30) Priority: 18.05.2016 US 201615158243
(43) Date of publication of application: 22.11.2017
(73) Proprietor: Arthrex Inc, Naples, FL 34108-1945 (US)
(72) Inventor: Seese, Ronald, Naples, FL 34119 (US); Bowers, William, Westminster, CO 80234 (US)
(74) Representative: Westphal, Mussgnug & Partner Patentanwälte mbB

(56) References cited:
- DE-A1- 10 046 592
- US-A- 4 517 976
- US-A1- 2003 158 546
- US-B1- 6 547 786

## Description

### FIELD OF THE DISCLOSURE

The present disclosure generally relates to electrosurgical devices, and more particularly to electrosurgical devices powering ablation devices usable in surgical procedures.

### BACKGROUND

Ablation devices have been used to remove tissue within patients in a variety of medical procedures. When in use, ablation devices are often operated throughout a range of power settings. Higher power settings are often used to remove tissue via vaporization and lower settings are used for desiccation. Some ablation devices include aspiration systems to remove fluid or particulates, or both, from a surgery site within a patient. Conventional aspiration systems remove fluid and particulates from a surgery site at the same aspiration rate regardless of the power setting of the active electrode. An aspiration rate large enough to properly remove material generated at a high power setting for an active electrode is too large of an aspiration rate for a low power setting for the active electrode. In particular, the aspiration rate corresponding with the high power setting interferes with a plasma field or ionized gas vapor layer at the active electrode generated at a low power setting such as by pulling the plasma field away from the active electrode, thus affecting the behavior of the plasma field. An electrosurgical system according to the preamble of claim 1 is known from US4517976.

### SUMMARY

The invention is defined in claim 1. Any methods disclosed hereinafter do not form part of the scope of the invention. An electrosurgical system including an electrosurgical unit with amplitude modulated output for ablation surgical devices, whereby the electrosurgical unit generates a signal in either a high mode or low mode, both of which are greater than zero, is disclosed. In at least one embodiment, the electrosurgical unit may be configured such that the power delivered to an electrode assembly of an ablation device in electrical communication with the electrosurgical unit is controlled by varying the duration or the intensity of power delivered during the high and low modes, or both. In another embodiment, the duration of the high mode may remain constant while the duration of the low mode may vary in order to vary the power output from the electrosurgical unit.

In at least one embodiment, the electrosurgical system may include an ablation device formed from an electrode assembly having an active electrode and a return electrode, wherein the active electrode is insulated from the return electrode. The electrosurgical system may be configured as a bipolar configuration, and in an alternative embodiment, may be a monopolar configuration. The electrosurgical system may include an electrosurgical unit configured to power the electrode assembly by generating a radio frequency output to drive the electrode assembly. The electrosurgical unit may be configured to generate an amplitude modulated waveform in which the waveform operates in a high mode or in a low mode, whereby both the high mode and the low mode are greater than zero amps. The electrosurgical unit may be configured to power the electrode assembly by generating the radio frequency output to drive the electrode assembly with the high mode continuously at a fixed time or the low mode at a variable time. The electrosurgical unit may be configured to power the electrode assembly by generating the radio frequency output to drive the electrode assembly with the high mode continuously at the fixed time or the low mode at the variable time at more than 0 Amps. In another embodiment, the electrosurgical unit may be configured to power the electrode assembly by generating the radio frequency output to drive the electrode assembly with the high mode continuously at the fixed time or the low mode at the variable time at more than 0.75 Amps. The electrosurgical unit may be configured to power the electrode assembly by generating a radio frequency output to drive the electrode assembly with the low mode at a variable time that is a function of impedance at a surgical site. In another embodiment, the electrosurgical unit may generate a radio frequency output with a variable time high mode and a fixed time low mode. In yet another embodiment, the electrosurgical unit may generate a radio frequency output with a variable time high mode and a variable time low mode.

The electrosurgical system may include a RF voltage sensor module and a RF current sensor module, which are configured to monitor, during each high mode, an RF signal generated by the electrosurgical unit to determine a voltage and a current, respectively, delivered to a surgical site via the electrode assembly. The electrosurgical system may also include one or more microcontrollers configured to communicate with the RF voltage current sensor module to determine impedance at a surgical site by dividing voltage by current at the electrosurgical unit. The electrosurgical system may also include one or more DC microcontrollers configured to use the impedance to determine how to deliver the RF output power to a surgical site by applying the impedance to a voltage, current and power curve. The impedance may be applied to the duration of the low mode to reduce the power defined by the VIP curve to a level that produces the appropriate RMS power. The DC microcontroller may be configured to store multiple voltage, current, power curves, whereby each voltage, current, power curve corresponds to a power setting on the ablation device. By determining the impedance, the DC microcontroller may work with the VIP curve and adjust the RMS power by appropriately adjusting the low mode time to adjust the output power.

The electrosurgical unit may be configured to power the electrode assembly by generating the radio frequency output to drive the electrode assembly with the high mode continuously at a fixed time of between about 15 and 40 milliseconds. In at least one embodiment, the electrosurgical unit may be configured to power the electrode assembly by generating the radio frequency output to drive the electrode assembly with the high mode continuously at a fixed time of about 25 milliseconds.

A method of controlling power deliver in an electrosurgical system may include receiving power in the electrosurgical system, whereby the electrosurgical system may include an ablation device formed from an electrode assembly having an active electrode and a return electrode, wherein the active electrode is insulated from the return electrode, and an electrosurgical unit configured to power the electrode assembly by generating a radio frequency output to drive the electrode assembly. The method may also include sending an amplitude modulated waveform from the electrosurgical unit to the electrode assembly, whereby the waveform operates in a high mode or in a low mode and both the high mode and the low mode are greater than zero amps, from the electrosurgical unit to the electrode assembly.

These and other embodiments are described in more detail below.

### BRIEF DESCRIPTION OF THE DRAWINGS

The accompanying drawings, which are incorporated in and form a part of the specification, illustrate embodiments of the presently disclosed invention and, together with the description, disclose the principles of the invention.
Figure 1 is a perspective view of an electrosurgical system including an electrosurgical unit with amplitude modulated output for ablation surgical devices, whereby the electrosurgical unit generates a signal in either a high mode or low mode, both of which are greater than zero, and an ablation device including active and return electrodes.
Figure 2 is a schematic diagram of one embodiment of the electrosurgical unit.
Figure 3 is a graph of output power versus load resistance, with the output power being the result of amplitude modulation.
Figure 4 is a flow chart of a method of using the electrosurgical system.
Figures 5-12 are graphs of different outputs from the electrosurgical unit corresponding to different impedances encountered in connection with target tissue in connection with the electrosurgical unit being set to an ablation 9 setting.
Figures 13-18 are graphs of different outputs from the electrosurgical unit corresponding to different resistances encountered in target tissue in connection with the electrosurgical unit being set to an ablation 6 setting.
Figures 19 is a graph of an output from the electrosurgical unit corresponding to a resistance of 10 ohms encountered in target tissue in connection with the electrosurgical unit being set to an a coagulation 1 setting.
Figures 20 is a graph of an output from the electrosurgical unit corresponding to a resistance of 10 ohms encountered in target tissue in connection with the electrosurgical unit being set to an a coagulation 2 setting.

### DETAILED DESCRIPTION

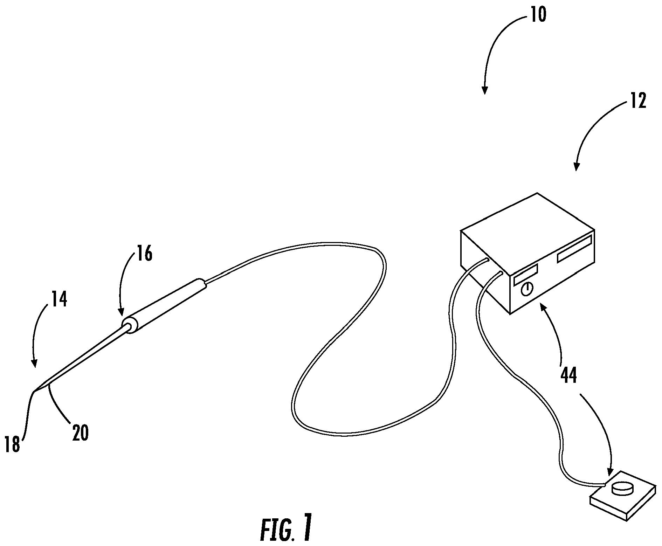

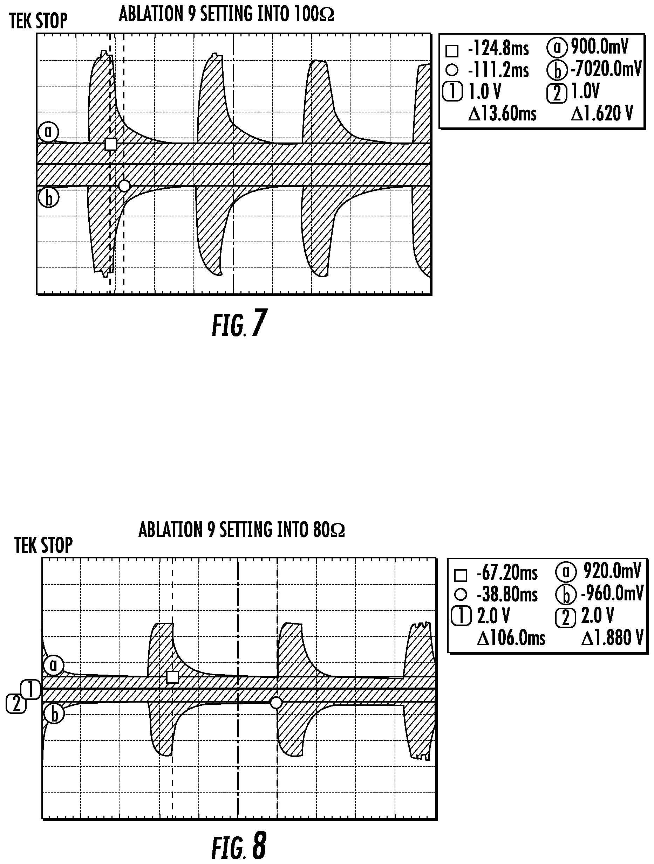

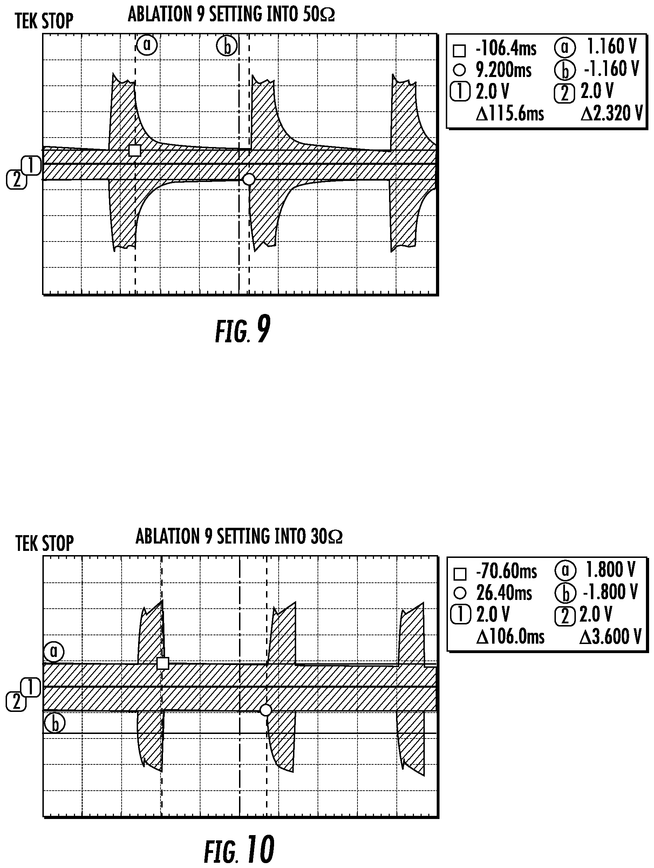

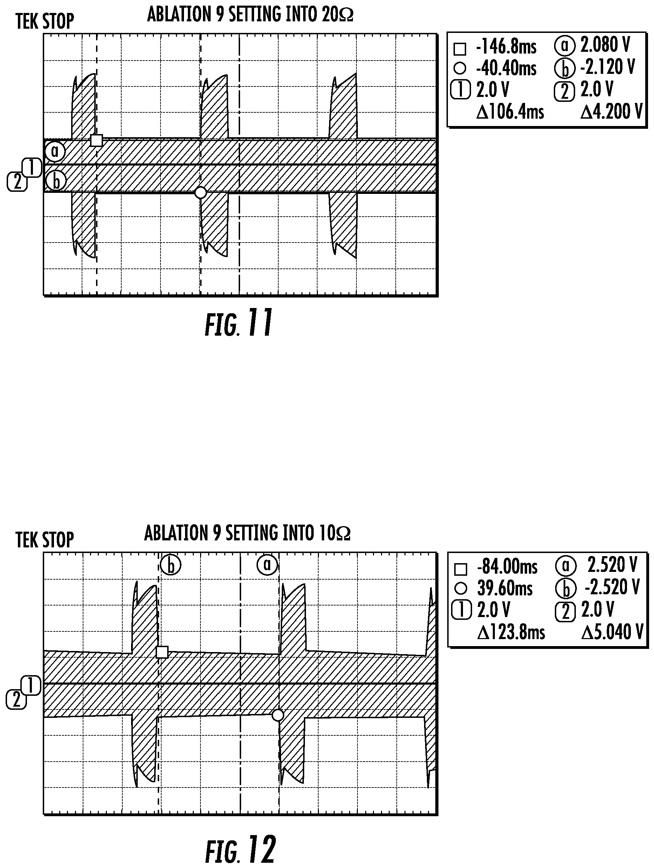

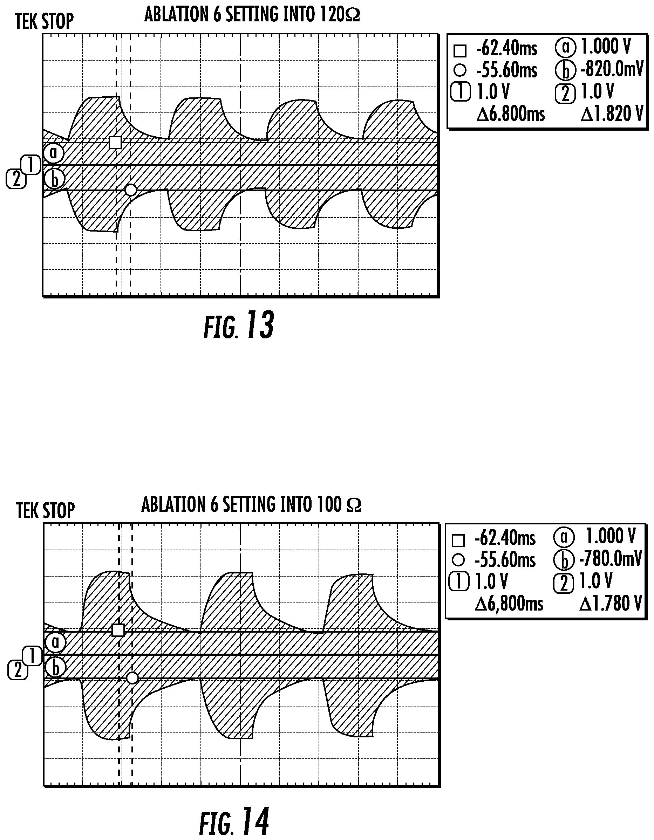

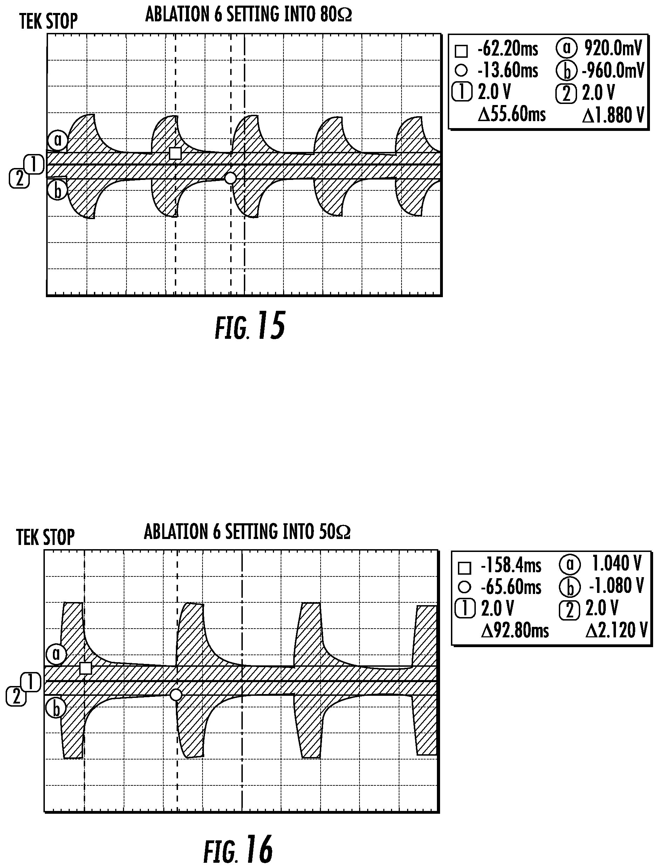

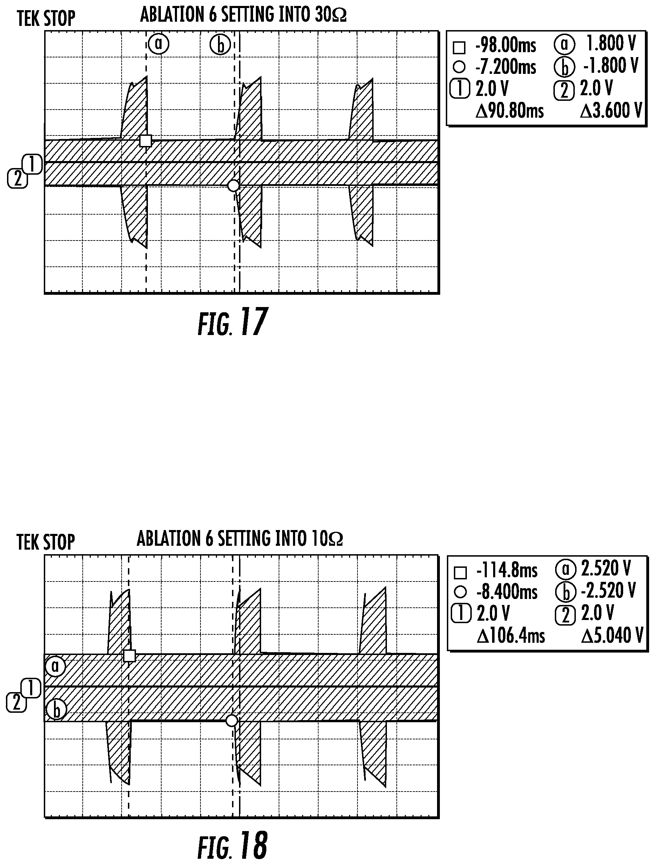

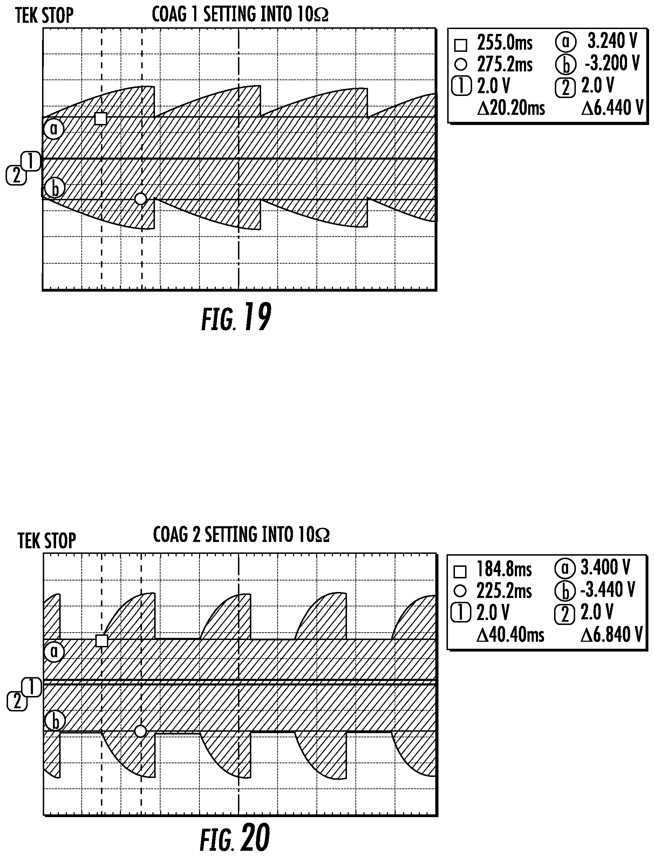

As shown in Figures 1-20, an electrosurgical system 10 including an electrosurgical unit 12 with amplitude modulated output for ablation surgical devices, whereby the electrosurgical unit 12 generates a signal in either a high mode or low mode, both of which are greater than zero, is disclosed. In at least one embodiment, the electrosurgical unit 12 may be configured such that the power delivered to an electrode assembly 14 of an ablation device 16 in electrical communication with the electrosurgical unit 12 is controlled by varying the duration or the intensity of power delivered during the high and low modes, or both. In another embodiment, the duration of the high mode may remain constant while the duration of the low mode may vary to vary the power output from the electrosurgical unit 12.

In at least one embodiment, the electrosurgical system 10 may be formed from an ablation device 16 formed from an electrode assembly 14 having an active electrode 18 and a return electrode 20. The active electrode 18 may be insulated from the return electrode 20. The electrosurgical system 10 may include an electrosurgical unit 12 configured to power the electrode assembly 14 by generating a radio frequency (RF) output to drive the electrode assembly 14. The electrosurgical unit 12 may be configured to genera an amplitude modulated waveform in which the waveform operates in a high mode or in a low mode, whereby both the high mode and the low mode are greater than zero amps. The electrosurgical unit 12 may be configured to power the electrode assembly 14 by generating the radio frequency output to drive the electrode assembly 14 with the high mode continuously at a fixed time or the low mode at a variable time. The electrosurgical unit may be configured to power the electrode assembly 14 by generating the radio frequency output to drive the electrode assembly 14 with the high mode continuously at the fixed time or the low mode at the variable time at more than zero Amps. In at least one embodiment, the electrosurgical unit may be configured to power the electrode assembly 14 by generating the radio frequency output to drive the electrode assembly 14 with the high mode continuously at the fixed time or the low mode at the variable time at more than 0.75 Amps. The electrosurgical unit 12 may be configured to power the electrode assembly 14 by generating a radio frequency output to drive the electrode assembly 14 with the low mode at a variable time that is a function of impedance at a surgical site.

The electrosurgical system 10 may also include a RF voltage sensor module 42 and a RF current sensor module 43 configured to monitor, during each high mode, an RF signal generated by the electrosurgical unit 12 to determine a voltage and a current, respectively, delivered to a surgical site via the electrode assembly 14. The electrosurgical system 10 may also one or more microcontrollers 24 configured to communicate with the RF voltage current sensor module 42 to determine impedance at a surgical site by dividing voltage by current at the electrosurgical unit 12. In at least one embodiment, the microcontroller 24 may be a DC microcontroller. The DC microcontroller 24 may be configured to use the impedance to determine how to deliver the RF output power to a surgical site by applying the impedance to a voltage, current and power curve. The DC microcontroller 24 may be is configured to store multiple voltage, current, power curves (VIPs) 26, whereby each voltage, current, power curve 26 corresponds to a power setting 28 on the ablation device 16. The electrosurgical unit 12 may be configured to power the electrode assembly 14 by generating the radio frequency output to drive the electrode assembly 14 with the high mode continuously at a fixed time of between about 15 and 40 milliseconds. In at least one embodiment, the electrosurgical unit 12 may be configured to power the electrode assembly 14 by generating the radio frequency output to drive the electrode assembly 14 with the high mode continuously at a fixed time of about 25 milliseconds.

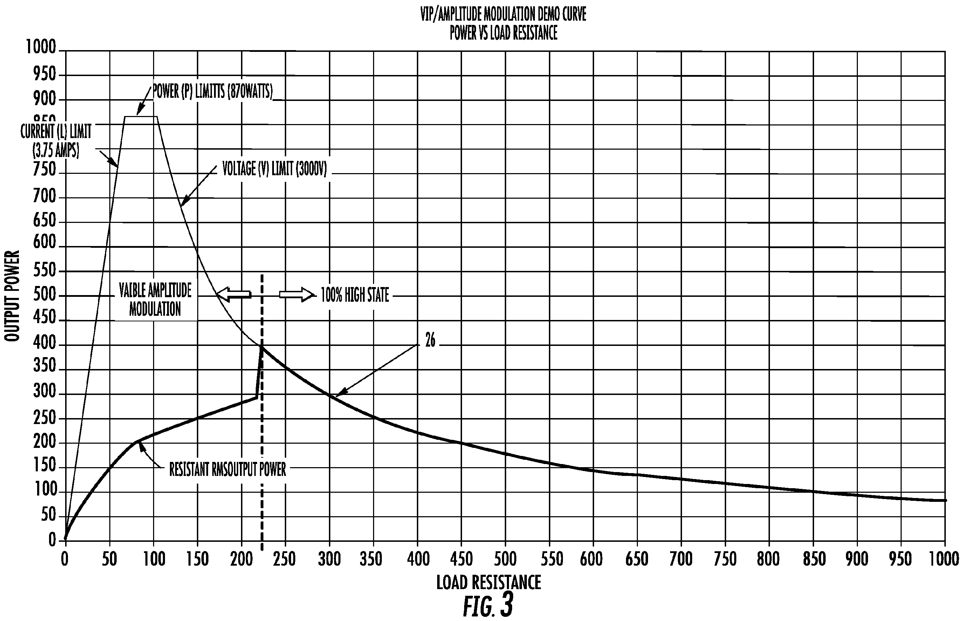

In at least one embodiment, the electrosurgical unit 12 may be a generator that provides a RF output to drive a bipolar electrosurgical ablation device 16 through the application of an electric current conducted using two electrodes of the ablation device 16. The electrosurgical unit 12 may generate an RF output having a square wave at about 100 kHz or other appropriate output. The electrosurgical unit 12 may include one or more voltage, current, power curves 26, as shown in Figure 3, representing the results from a power curve algorithm. The electrosurgical unit 12 may use the voltage, current, power curves 26 to determine the amount of power to send from the electrosurgical unit 12 to the electrode assembly 14. The electrosurgical unit 12 may adjust power output through a feedback loop based on calculated output impedance.

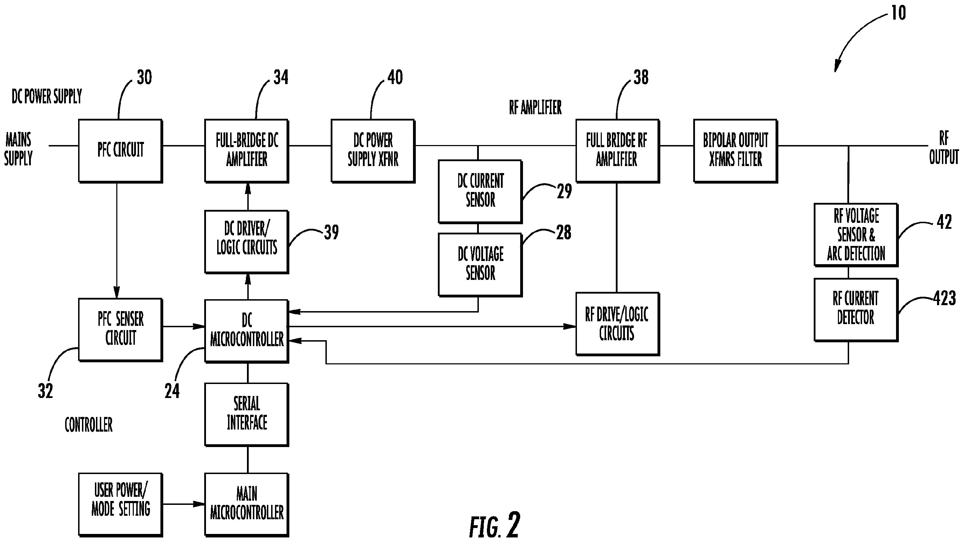

In at least one embodiment of the electrosurgical system 10, as shown in Figure 2, AC power is supplied to the electrosurgical unit 12 and directly applied to the power factor correction ("PFC") off-line converter 30 through a network of fuses, inrush limiters and a power ON/OFF switch. The PFC converter 30 may provide an active power factor correction for the power supply and may output a voltage to a PFC sense circuit 32 and a full bridge DC amplifier 34. The electrosurgical unit 12 may include an isolation digital optocoupler to isolate digital signals that represent a PFC voltage. The isolation digital optocoupler may be used by the DC microcontroller 24 to ensure proper PFC voltage. The full-bridge DC amplifier 34 may use the output voltage of the PFC sense circuit 32 to control the RF output power by adjusting the DC output voltage applied to an RF amplifier 38. In at least one embodiment, the RF amplifier may be a full-bridge, Class D amplifier operating at 100 kHz, squarewave output waveform, which is driven via the DC microcontroller 24.

A drive circuit 36 drives one or more transistors, such as, but not limited to upper and lower power transistors, in the full-bridge DC amplifier 34, whose output may be applied to a DC power supply transformer 40. The DC power supply transformer 40 may provide isolation between the AC main circuits and the high-voltage intermediate circuits. When the RF amplifier 38 begins to deliver RF energy to a surgical site, the electrosurgical unit 12, such as, but not limited, to the PFC circuit 28 sense the DC voltage and current and feed back into the DC microcontroller 24. The DC microcontroller 24 may control the output power using a controller algorithm and RMS voltage and current produced by RF voltage/current and arc detection circuitry 42. In at least one embodiment, the DC microcontroller 24 may control the output power via software contained in the DC microcontroller 24. In at least one embodiment, the microcontroller 24 of the electrosurgical unit 12 uses a control algorithm to properly control the RF output characteristics of the electrosurgical unit 12 to provide a stable RF output to a surgical site on or within a patient.

In at least one embodiment, the ablation device 16 may include controls 44 for controlling operating of the electrode assembly 14. In particular, the controls 44 may include controls for ablation mode, coagulation mode and power levels. In at least one embodiment, the controls 44 may be physical buttons for ablation mode, coagulation mode and power levels. In other embodiments, the controls 44 may be formed from other appropriate devices. The power level control may include power level settings from 1 to 9. The electrosurgical system 10 may also include one or more footswitches to control the on/off settings of the electrosurgical unit 12. As such, a surgeon using the ablation device 16 may be able to prioritize operation and control between the ablation device 16 and footswitch.

In at least one embodiment, the electrosurgical system 10 may be configured to operate in two different modes of operation: ablation and coagulation. The ablation mode may have two different operating characteristics: continuous waveform and amplitude modulated waveform. The amplitude modulated waveform may include a High mode continuously for a fixed amount of time and a Low mode as a variable time that is a function of the impedance at the surgical site. During each High mode, the RF voltage/current sense circuit 42 may monitor the actual current and voltage being delivered to the surgical site. The DC microcontroller 24 may obtain the impedance at the surgical site by dividing the voltage by the current. The microcontroller 24 applies the measured impedance to a voltage, current, power (VIP) curve to define how the RF output power is to be delivered to the surgical site. Each power setting, such as power settings 1-9, is matched with a different VIP power curve. Each VIP power curve is defined to match the output characteristics of the ablation device 16. An exemplary VIP power curve is shown in Figure 3.

As shown in Figure 3, the VIP power curve is defined by three different modes of operation: voltage limit (V), current limit (I), and power limit (P). The DC microcontroller 24 determines what mode of operation (*i.e.,* V, I, or P) the electrosurgical unit 12 should be operating in based upon the power setting and the measured impedance at the surgical site. For example, if the sensed output impedance is greater than approximately 100 ohms (Ω), the microcontroller 24 will cause the electrosurgical unit 12 to generate RF power to the ablation device such that the ablation device 16 will operate in a constant voltage mode (*e.g.,* 300 V). As a result, the microcontroller 24 will regulate the output characteristics to provide a constant voltage to a surgical site. If the sensed output impedance is less than approximately 65 Ω, the microcontroller 24 will cause the electrosurgical unit 12 to generate RF power to the ablation device 16 such that the ablation device 16 will operate in a constant current mode (*e.g.,* 3.75 Amps (A)). The electrosurgical unit 12 may be configured to operate in a constant power mode (*e.g.,* 870 watts (W)) between these two impedances of 65 Ω and 100 Ω. Accordingly, during operation, the electrosurgical unit 12 is constantly adjusting its operating mode and output characteristics based on the measured impedance.

An impedance switching point is defined for each power setting. The impedance switching point is a point at which the output operates in the continuous waveform or the amplitude modulated waveform for the ablation mode as discussed above. As shown in the exemplary VIP curve in Figure 3, the switching point is defined at approximately 215 Ω. Thus, for impedances greater than 215 Ω, the electrosurgical unit 12 will generate an output as a continuous waveform, and for impedances less than 215 Ω, the electrosurgical unit 12 will generate an output as an amplitude modulated waveform. The full bridge DC amplifier 34 may perform the amplitude modulation switching at a predefined rate as a function of the impedance at the surgical site. In particular, in at least one embodiment, the amplitude modulation begins with a High mode with a duration, such as between about 10 and about 40 milliseconds (ms). In at least one embodiment, the High mode may have a duration of 25 ms. As the DC amplifier 34 turns to ON, energy is delivered to the surgical site through the full bridge RF amplifier 34.

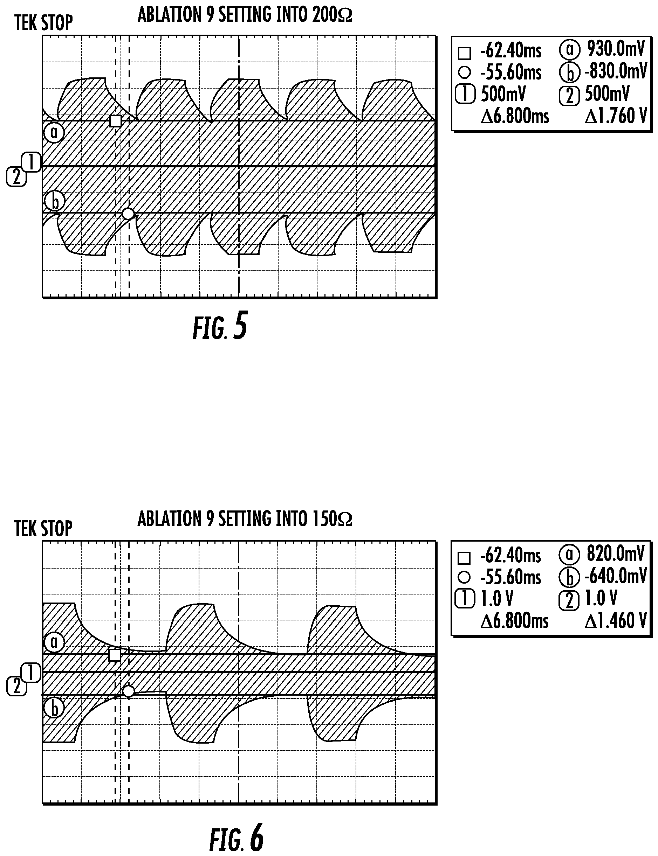

The DC microcontroller 24 may use energy sensed by the voltage sense circuit 28 and current sense circuit 29 (*i.e.,* the measured impedance) to define where the electrosurgical unit 12 should be operating in relation to the VIP power curve, such as in one of the regions with a voltage limit, current limit, or power limit. The DC microcontroller 24 may continuously monitor where the electrosurgical unit 12 should be operating in relation to the VIP power curve and may maintain a condition throughout a 25 ms timeframe. At the end of a 25 ms duration, the DC microcontroller 24 may determine the output impedance and may determine whether the output characteristic generated from the electrosurgical unit 12 as an RF signal should be transmitted in a continuous waveform or an amplitude modulated waveform. If the electrosurgical unit 12 is operating in the amplitude modulated waveform, the DC microcontroller 24 may determine an appropriate duration to operate in the Low mode that, when combined with the duration of the High mode, will generate a variable amplitude modulation (HIGH amplitude modulated duration / (HIGH amplitude modulated duration + LOW amplitude modulated duration) ratio, where the High duration time is always a fixed time, such as, but not limited to 25 ms). By combining the amplitude modulation with the VIP power curve, such as with the DC microcontroller 24, a desired RMS output power can be defined. In particular, for a given power setting, as shown in the exemplary VIP curve in Figure 3, the DC microcontroller 24 continuously monitors and adjusts the output characteristics to ensure the desired RMS output characteristics are maintained. As shown in Figures 5-20, the curves labeled "Resultant RMS Output Power" shows the resultant desired RMS output power for the amplitude modulated waveform. Each power setting has different characteristics that define the resultant RMS output power, but the multi-functional control system operates in the same manner.

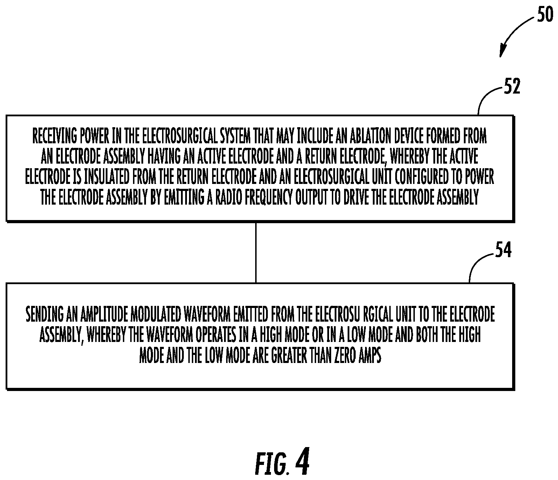

As shown in Figure 4, a method 50 for controlling power delivered in the electrosurgical system 10 is disclosed for using the electrosurgical system 10. The method 50 may include at 52 receiving power in the electrosurgical system 10 configured as set forth above. In particular, the method 50 may include an ablation device 16 formed from an electrode assembly 14 having an active electrode 18 and a return electrode 30, whereby the active electrode 18 is insulated from the return electrode 30 and an electrosurgical unit 12 configured to power the electrode assembly 14 by generating a radio frequency output to drive the electrode assembly 14. The method 50 may include at 54 sending an amplitude modulated waveform generated from the electrosurgical unit 12 to the electrode assembly 14, whereby the waveform operates in a high mode or in a low mode and both the high mode and the low mode are greater than zero amps.

The step at 54 of sending the amplitude modulated waveform from the electrosurgical unit 12 to the electrode assembly 14 may include sending the amplitude modulated waveform, whereby the amplitude modulated waveform is a radio frequency output configured to drive the electrode assembly with the high mode continuously at a fixed time or the low mode at a variable time. The step at 54 of sending the amplitude modulated waveform from the electrosurgical unit 12 to the electrode assembly 14 may include sending the amplitude modulated waveform, whereby the amplitude modulated waveform is formed from both the high mode operating continuously at the fixed time or the low mode operating at the variable time at more than 0.75 Amps. The step at 54 of sending the amplitude modulated waveform from the electrosurgical unit 12 to the electrode assembly 14 may include sending the amplitude modulated waveform, whereby the amplitude modulated waveform is a radio frequency output configured to drive the electrode assembly 14 with the low mode at a variable time that is a function of impedance at a surgical site.

The step at 54 of sending the amplitude modulated waveform from the electrosurgical unit 12 to the electrode assembly 14 may include determining a current and a voltage delivered to a surgical site via the electrode assembly with use of a RF voltage current sensor module 42 configured to monitor, during each high mode, an RF signal generated by the electrosurgical unit 12. The step at 54 of sending the amplitude modulated waveform from the electrosurgical unit 12 to the electrode assembly 14 may include determining impedance at a surgical site via at least one microcontroller 24 configured to communicate with the RF voltage current sensor module 42 to determine impedance at a surgical site by dividing voltage by current at the electrosurgical unit 12.

The step at 54 of sending the amplitude modulated waveform from the electrosurgical unit 12 to the electrode assembly 14 may include determining impedance at a surgical site via the DC microcontroller 24 using the impedance to determine how to deliver the RF output power to a surgical site by applying the impedance to a voltage, current and power curve. The step at 54 of sending the amplitude modulated waveform from the electrosurgical unit 12 to the electrode assembly 14 may include determining impedance at a surgical site via the DC microcontroller 24 accessing multiple voltage, current, power curves, whereby each voltage, current, power curve corresponds to a power setting on the ablation device 16. The step at 54 of sending the amplitude modulated waveform from the electrosurgical unit 12 to the electrode assembly 14 may include generating the radio frequency output to drive the electrode assembly 14 with the high mode continuously at a fixed time of between about 15 and 40 milliseconds.

The foregoing is provided for purposes of illustrating, explaining, and describing embodiments of this invention. Modifications and adaptations to these embodiments will be apparent to those skilled in the art and may be made without departing from the scope of this invention.

## Claims

1. An electrosurgical system, comprising:
an ablation device formed from an electrode assembly having an active electrode and a return electrode, wherein the active electrode is insulated from the return electrode; and
an electrosurgical unit configured to power the electrode assembly by generating a radio frequency output to drive the electrode assembly, wherein the electrosurgical unit is configured to generate an amplitude modulated waveform in which the waveform operates in a high mode or in a low mode, whereby both the high mode and the low mode are greater than zero amps,
**characterized in that** the electrosurgical unit is configured to generate and supply to the electrode assembly a continuous waveform when sensed impedance at the electrode assembly is greater than a threshold value and wherein the electrosurgical unit is configured to generate and supply to the electrode assembly the amplitude modulated waveform when sensed impedance at the electrode assembly is less than or equal to the threshold value.

2. The electrosurgical system of claim 1, wherein the electrosurgical unit is configured to power the electrode assembly by generating the radio frequency output to drive the electrode assembly with the high mode continuously at a variable time or the low mode at a variable time.

3. The electrosurgical system of claim 1, wherein the electrosurgical unit is configured to power the electrode assembly by generating the radio frequency output to drive the electrode assembly with the high mode continuously at a variable time or the low mode at a fixed time.

4. The electrosurgical system of claim 1, wherein the electrosurgical unit is configured to power the electrode assembly by generating the radio frequency output to drive the electrode assembly with the high mode continuously at a fixed time or the low mode at a variable time.

5. The electrosurgical system of claim 1, wherein the electrosurgical unit is configured to power the electrode assembly by generating the radio frequency output to drive the electrode assembly with the high mode continuously at the fixed time or the low mode at the variable time at more than 0.75 Amps.

6. The electrosurgical system of claim 1, wherein the electrosurgical unit is configured to power the electrode assembly by generating a radio frequency output to drive the electrode assembly with the low mode at a variable time that is a function of impedance at a surgical site.

7. The electrosurgical system of claim 1, further comprising a RF voltage sensor module configured to monitor, during each high mode, an RF signal generated by the electrosurgical unit to determine voltage delivered to a surgical site via the electrode assembly.

8. The electrosurgical system of claim 7, further comprising at least one microcontroller configured to communicate with the RF voltage sensor module to determine impedance at a surgical site by dividing voltage by current at the electrosurgical unit.

9. The electrosurgical system of claim 8, wherein the at least one microcontroller is a DC microcontroller configured to use the impedance to determine how to deliver the RF output power to a surgical site by applying the impedance to a voltage, current and power curve.

10. The electrosurgical system of claim 9, wherein the at least one DC microcontroller is configured to store multiple voltage, current, power curves, whereby each voltage, current, power curve corresponds to a power setting on the ablation device.

11. The electrosurgical system of claim 1, wherein the electrosurgical unit is configured to power the electrode assembly by generating the radio frequency output to drive the electrode assembly with the high mode continuously at a fixed time of between about 15 and 40 milliseconds.

## Patentansprüche

1. Elektrochirurgisches System, umfassend:
eine Ablationsvorrichtung, welche aus einer Elektrodenanordnung gebildet ist, die eine aktive Elektrode und eine Gegenelektrode aufweist, wobei die aktive Elektrode von der Gegenelektrode isoliert ist; und
eine elektrochirurgische Einheit, welche dazu ausgebildet ist, die Elektrodenanordnung mit Strom zu versorgen, indem sie einen Radiofrequenzausgang erzeugt, um die Elektrodenanordnung anzusteuern, wobei die elektrochirurgische Einheit dazu ausgebildet ist, eine amplitudenmodulierte Wellenform zu erzeugen, in der die Wellenform in einem hohen Betrieb oder in einem niedrigen Betrieb arbeitet, wobei der hohe Betrieb und der niedrige Betrieb größer als null Ampere sind,
**dadurch gekennzeichnet, dass** die elektrochirurgische Einheit dazu ausgebildet ist, eine kontinuierliche Wellenform zu erzeugen und der Elektrodenanordnung zu speisen, wenn eine gemessene Impedanz an der Elektrodenanordnung größer als ein Schwellenwert ist, und wobei die elektrochirurgische Einheit dazu ausgebildet ist, die amplitudenmodulierte Wellenform zu erzeugen und der Elektrodenanordnung zu speisen, wenn eine gemessene Impedanz an der Elektrodenanordnung kleiner als oder gleich dem Schwellenwert ist.

2. Elektrochirurgisches System gemäß Anspruch 1, wobei die elektrochirurgische Einheit dazu ausgebildet ist, die Elektrodenanordnung mit Strom zu versorgen, indem sie den Radiofrequenzausgang erzeugt, um die Elektrodenanordnung mit dem hohen Betrieb kontinuierlich zu einer variablen Zeit oder mit dem niedrigen Betrieb zu einer variablen Zeit anzusteuern.

3. Elektrochirurgisches System gemäß Anspruch 1, wobei die elektrochirurgische Einheit dazu ausgebildet ist, die Elektrodenanordnung mit Strom zu versorgen, indem sie den Radiofrequenzausgang erzeugt, um die Elektrodenanordnung mit dem hohen Betrieb kontinuierlich zu einer variablen Zeit oder mit dem niedrigen Betrieb zu einer festgelegten Zeit anzusteuern.

4. Elektrochirurgisches System gemäß Anspruch 1, wobei die elektrochirurgische Einheit dazu ausgebildet ist, die Elektrodenanordnung mit Strom zu versorgen, indem sie den Radiofrequenzausgang erzeugt, um die Elektrodenanordnung mit dem hohen Betrieb kontinuierlich zu einer festgelegten Zeit oder mit dem niedrigen Betrieb zu einer variablen Zeit anzusteuern.

5. Elektrochirurgisches System gemäß Anspruch 1, wobei die elektrochirurgische Einheit dazu ausgebildet ist, die Elektrodenanordnung mit Strom zu versorgen, indem sie den Radiofrequenzausgang erzeugt, um die Elektrodenanordnung mit dem hohen Betrieb kontinuierlich zu der festgelegten Zeit oder mit dem niedrigen Betrieb zu der variablen Zeit bei mehr als 0,75 Ampere anzusteuern.

6. Elektrochirurgisches System gemäß Anspruch 1, wobei die elektrochirurgische Einheit dazu ausgebildet ist, die Elektrodenanordnung mit Strom zu versorgen, indem sie den Radiofrequenzausgang erzeugt, um die Elektrodenanordnung mit dem niedrigen Betrieb zu einer variablen Zeit, die eine Funktion der Impedanz an einer Eingriffsstelle ist, anzusteuern.

7. Elektrochirurgisches System gemäß Anspruch 1, weiter umfassend ein RF Spannungssensormodul, das dazu ausgebildet ist, während jedes hohen Betriebs, ein RF Signal, das durch die elektrochirurgische Einheit erzeugt wird, zu überwachen, um eine Spannung, die an eine Eingriffsstelle über die Elektrodenanordnung geliefert wird, zu bestimmen.

8. Elektrochirurgisches System gemäß Anspruch 7, weiter umfassend mindestens einen Mikrocontroller, der dazu ausgebildet ist, mit dem RF Spannungssensormodul zu kommunizieren, um eine Impedanz an einer Eingriffsstelle zu bestimmen, indem die Spannung durch den Strom an der elektrochirurgischen Einheit dividiert wird.

9. Elektrochirurgisches System gemäß Anspruch 8, wobei der mindestens eine Mikrocontroller ein DC Mikrocontroller ist, der dazu ausgebildet ist, die Impedanz zu verwenden, um zu bestimmen, wie die RF Ausgangsleistung an eine Eingriffsstelle geliefert werden soll, indem die Impedanz auf eine Spannungs-, Strom- und Leistungskurve angewandt wird.

10. Elektrochirurgisches System gemäß Anspruch 9, wobei der mindestens eine DC Mikrocontroller dazu ausgebildet ist, mehrere Spannungs-, Strom- , Leistungskurven zu speichern, wobei jede Spannungs-, Strom-, Leistungskurve einer Leistungseinstellung auf der Ablationsvorrichtung entspricht.

11. Elektrochirurgisches System gemäß Anspruch 1, wobei die elektrochirurgische Einheit dazu ausgebildet ist, die Elektrodenanordnung mit Strom zu versorgen, indem sie den Radiofrequenzausgang erzeugt, um die Elektrodenanordnung mit dem hohen Betrieb kontinuierlich zu einer festgelegten Zeit von zwischen etwa 15 und 40 Millisekunden anzusteuern.

## Revendications

1. Système électrochirurgical, comprenant :
un dispositif d'ablation formé à partir d'un ensemble d'électrode comportant une électrode active et une électrode de retour, l'électrode active étant isolée de l'électrode de retour ; et
une unité électrochirurgicale configurée pour alimenter l'ensemble d'électrode en générant une sortie de fréquence radio de manière à commander l'ensemble d'électrode, l'unité électrochirurgicale étant configurée pour générer une forme d'onde modulée en amplitude dans laquelle la forme d'onde fonctionne dans un mode haut ou dans un mode bas, le mode haut et le mode bas étant supérieurs à zéro ampère,
**caractérisé en ce que** l'unité électrochirurgicale est configurée pour générer et fournir à l'ensemble d'électrode une forme d'onde continue quand une impédance mesurée au niveau de l'ensemble d'électrode est supérieure à une valeur seuil et dans lequel l'unité électrochirurgicale est configurée pour générer et fournir à l'ensemble d'électrode la forme d'onde modulée en amplitude quand une impédance mesurée au niveau de l'ensemble d'électrode est inférieure ou égale à la valeur seuil.

2. Système électrochirurgical selon la revendication 1, dans lequel l'unité électrochirurgicale est configurée pour alimenter l'ensemble d'électrode en générant la sortie de fréquence radio de manière à commander l'ensemble d'électrode avec le mode haut de manière continue à un temps variable ou le mode bas à un temps variable.

3. Système électrochirurgical selon la revendication 1, dans lequel l'unité électrochirurgicale est configurée pour alimenter l'ensemble d'électrode en générant la sortie de fréquence radio de manière à commander l'ensemble d'électrode avec le mode haut de manière continue à un temps variable ou le mode bas à un temps fixe.

4. Système électrochirurgical selon la revendication 1, dans lequel l'unité électrochirurgicale est configurée pour alimenter l'ensemble d'électrode en générant la sortie de fréquence radio de manière à commander l'ensemble d'électrode avec le mode haut de manière continue à un temps fixe ou le mode bas à un temps variable.

5. Système électrochirurgical selon la revendication 1, dans lequel l'unité électrochirurgicale est configurée pour alimenter l'ensemble d'électrode en générant la sortie de fréquence radio de manière à commander l'ensemble d'électrode avec le mode haut de manière continue au temps fixe ou le mode bas au temps variable à plus de 0,75 ampères.

6. Système électrochirurgical selon la revendication 1, dans lequel l'unité électrochirurgicale est configurée pour alimenter l'ensemble d'électrode en générant la sortie de fréquence radio de manière à commander l'ensemble d'électrode avec le mode bas à un temps variable qui est une fonction de l'impédance à un site opératoire.

7. Système électrochirurgical selon la revendication 1, comprenant en outre un module de capteur de tension RF configuré pour surveiller, pendant chaque mode haut, un signal RF généré par l'unité électrochirurgicale afin de déterminer la tension délivrée au site opératoire via l'ensemble d'électrode.

8. Système électrochirurgical selon la revendication 7, comprenant en outre au moins un microcontrôleur configuré pour communiquer avec le module de capteur de tension RF afin de déterminer l'impédance au niveau du site opératoire en divisant la tension par le courant au niveau de l'unité électrochirurgicale.

9. Système électrochirurgical selon la revendication 8, dans lequel le ou les microcontrôleurs sont des microcontrôleurs cc configurés pour utiliser l'impédance pour déterminer comment délivrer la puissance de sortie RF au site opératoire en appliquant l'impédance à une courbe de tension, de courant et de puissance.

10. Système électrochirurgical selon la revendication 9, dans lequel le ou les microcontrôleurs cc sont configurés pour stocker des courbes de tension, de courant, de puissance, chaque courbe de tension, de courant, de puissance correspondant à un réglage de puissance sur le dispositif d'ablation.

11. Système électrochirurgical selon la revendication 1, dans lequel l'unité électrochirurgicale est configurée pour alimenter l'ensemble d'électrode en générant la sortie de fréquence radio de manière à commander l'ensemble d'électrode avec le mode haut de manière continue à un temps fixe compris entre environ 15 et 40 millisecondes.
